# EUROPÄISCHE PATENTSCHRIFT

(11) **EP 1 558 364 B1**
(45) Veröffentlichungstag und Bekanntmachung des Hinweises auf die Patenterteilung: **24.03.2010**
(21) Anmeldenummer: 03772249.3
(22) Anmeldetag: 23.10.2003
(51) Int. Cl.: B01D 53/14, C07D 301/12, C07D 301/32

(54) **VERFAHREN ZUR KONTINUIERLICHEN RÜCKFÜHRUNG DES BEI DER OXIDATION VON OLEFINEN MIT HYDROPEROXIDEN NICHT UMGESETZTEN OLEFINS MITTELS LÖSUNGSMITTELWÄSCHE**
METHOD FOR CONTINUOUSLY RETURNING AN OLEFIN WHICH IS NOT REACTED WITH HYDROPEROXIDES DURING OXIDATION BY OLEFINS BY MEANS SOLVENT WASHING
PROCEDE DE RECYCLAGE EN CONTINU PAR LAVAGE AVEC DES SOLVANTS DE L'OLEFINE N'AYANT PAS REAGI LORS DE L'OXYDATION D'OLEFINES AVEC DES HYDROPEROXYDES

(30) Priorität: 23.10.2002 DE 10249379
(43) Veröffentlichungstag der Anmeldung: 03.08.2005
(73) Patentinhaber: BASF SE, 67056 Ludwigshafen (DE)
(72) Erfinder: BASSLER, Peter, 68519 Viernheim (DE); GÖBBEL, Hans-Georg, 67169 Kallstadt (DE); TELES, Joaquim-Henrique, 67166 Otterstadt (DE); RUDOLF, Peter, 68526 Ladenburg (DE)
(74) Vertreter: Fiesser, Gerold Michael
(86) Internationale Anmeldenummer: PCT/EP2003/011735
(87) Internationale Veröffentlichungsnummer: WO 2004/037390

(56) Entgegenhaltungen:
- EP-A- 0 583 828
- EP-A- 0 719 768
- WO-A-02/102496
- DE-B- 1 212 507
- US-A- 5 599 955

## Beschreibung

Die Erfindung betrifft ein Verfahren zur kontinuierlichen Rückführung des bei der Oxidation von Olefinen mit Hydroperoxiden zu Oxiranen nicht umgesetzten Olefins, wobei das Olefin aus dem bei der Oxidation entstehenden Abgasstrom zunächst in einem Lösungsmittel, das aus Kohlenwasserstoffen besteht, vorzugsweise Tetradekan, absorbiert, anschließend daraus desorbiert, eventuell von Aliphaten gereinigt, und in den Oxidationsprozess zurückgeführt wird, nämlich zur Rückführung des bei der Herstellung von Propenoxid eingesetzten Propens. Die Erfindung betrifft auch eine Vorrichtung, mit der das erfindungsgemäße Verfahren durchgeführt werden kann.

Bekanntlich nimmt bei der Oxidation von Olefinen mit Hydroperoxiden zu Oxiranen mit steigendem Olefinumsatz die Selektivität der Oxiranbildung zugunsten unerwünschter Nebenreaktionen deutlich ab. Um trotzdem eine hohe Selektivität von beispielsweise über 95 % erreichen zu können, werden deshalb insbesondere im technischen Maßstab diese Reaktionen vorzugsweise nur bis zu einem Olefinumsatz von ca. 85 bis 98 % betrieben.

Die EP 0 583 828 A2 betrifft ein Verfahren zur Direktoxidation von Ethylen. Dabei wird die Absorption und Desorption von Ethylen aus einem Argonspülgas in bzw. aus organischen Lösungsmitteln offenbart.

Die DE 12 12 507 beschreibt die Absorption und Desorption von Propen in organischen Lösungsmitteln. Dabei wird ein Verfahren zur Direktoxidation von Propen offenbart.

Die US 5,599,955 offenbart ein Verfahren zur Herstellung von Propen und von Propenoxid umfassend eine Auftrennung eines Propen/Propan-Gemischs. Eine Absorption und Desorption von Propen in einem Lösungsmittel wird in der US 5,599,955 nicht beschrieben.

WO 02/102496 betrifft ein Verfahren zur Riickgewinnung von brennbaren Komponenten aus einem Gasstrom, der die brennbaren Komponenten und Sauerstoff enthält, durch selektive Absorption der brennbaren Komponenten in einem Lösungsmittel. Insbesondere wird ein Verfahren zur Rückgewinnung von brennbaren Komponenten aus einem Austrittsgasstrom aus einer Oxidationsreaktion mit einer Peroxidkomponente offenbart, wobei sich aufgrund der Zersetzung des Peroxids während der Oxidationsreaktion Sauerstoff ansammelt.

WO 01/96271 A2betrifft ein Verfahren zur Herstellung von Acrolein und/oder Acrylsäure aus Propan und/oder Propen, wobei das Verfahren die Abtrennung von Propan und/oder Propen aus einem Propan und/oder Propen enthaltenden Gasgemisch durch Absorption in einem Absorptionsmittel, die Abtrennung des Propans und/oder Propens von dem Absorptionsmittel unter Erhalt eines Gases B und die Verwendung des erhaltenen Gases B für eine Oxidation von Propan und/oder Propen zu Acrolein und/oder Acrylsäure umfasst, wobei zwischen den letzten beiden Schritt keine heterogen katalysierte Dehydrierung von Propan ohne Zufuhr von Sauerstoff durchgeführt wird.

Es ist auch bereits bekannt, das nicht umgesetzte Olefin aus dem Reaktionsprozess zu isolieren und dann wieder in den Oxidationsprozess zurückzuführen.

So wurde ein Verfahren vorgeschlagen, bei dem ein Gasgemisch, bestehend aus dem Olefin und Sauerstoff, der aus der Zersetzungsreaktion des bei der Oxidation als Hydroperoxid verwendeten Wasserstoffperoxids stammt, abgetrennt, das Olefin in einem flüssigen Absorptionsmittel aus dem Gasgemisch absorbiert und dem Sauerstoff ein Inertgas in ausreichender Menge zugesetzt wird, um die Bildung entflammbarer Gaszusammensetzungen zu verhindern (EP-B 0 719 768 B1). In einer bevorzugten Ausführung wird dieses Verfahren zur Wiedergewinnung von Propen aus der Umsetzung mit Wasserstoffperoxid zu Propenoxid verwendet. Als Inertgas wird vorzugsweise Methan und als flüssiges Absorptionsmittel ein Gemisch, das Isopropanol und Wasser umfasst, eingesetzt. Weniger vorteilhaft können nach diesem Verfahren auch Kohlenwasserstoffe, wie Heptan und Oktan, sowie Methanol und Aceton verwendet werden.

Ein Nachteil des geschilderten Verfahrens ist jedoch darin zu sehen, dass der zur Absorption verwendeten Kolonne zusätzlich zum Abgasstrom ein weiteres Gas, insbesondere Methan, zugeführt werden muss. Durch diese Maßnahme soll aufgrund des Sauerstoffanteils im Gasgemisch die Ausbildung explosionsfähiger Gemische verhindert werden.

Auch wirkt sich bei diesem Verfahren die geringe Löslichkeit des Olefins im wasserhaltigen Isopropanol nachteilig aus. Beispielsweise müssen gemäß der Beschreibung Lösungsmittelgemische verwendet werden, die einen Gehalt an Wasser zwischen 30,6 Mol-% und 57,2 Mol-% aufweisen. Dieser hohe Wassergehalt beeinträchtigt die Löslichkeit des Olefins im Isopropanol. Deshalb müssen relativ große Mengen an Lösungsmittel eingesetzt werden, um das Olefin trotzdem durch Absorption aus dem Abgasstrom gewinnen zu können.

Es war daher die Aufgabe gestellt, ein verbessertes Verfahren zur Rückgewinnung des bei der Oxidation von Olefinen zu Oxiranen verwendeten Olefins bereitzustellen, das ohne die zusätzliche Einspeisung eines Inertgases in die Absorberkolonne auskommt, und mit dem eine effektivere Rückgewinnung des Olefins aus dem Abgasstrom möglich ist als beim Verfahren des Standes der Technik.

Diese Aufgabe konnte erfindungsgemäß durch ein Verfahren zur kontinuierlichen Rückführung des bei der Oxidation von Propen, welches als gesättigten Kohlenwasserstoff Propan enthält, mit Hydroperoxid zu Propenoxid nicht umgesetzten Propens, das im während der Oxidation entstehenden Abgasstrom enthalten ist, welcher Propan enthält, **dadurch gekennzeichnet, dass** es die Stufen (i) bis (iii) umfasst
(i) Abtrennung des Propens und Propans aus dem Abgasstrom durch Absorption in einem Kohlenwasserstoff,
(ii) Desorption des Propens und Propans aus dem Kohlenwasserstoff, wobei das Gemisch aus Propen und Propan bei einem Druck von 1 bis 3 bar in einer Destillationskolonne in flüssiger Form oder in einer Entspannungsverdampfung bei einem Druck von 1 bis 3 bar und einer Temperatur von 50 bis 100 °C in dampfförmiger Form abgetrennt wird;
(iii) Rückführung des in Stufe (ii) erhaltenen Propens in den Oxidationsprozess,
wobei das nach der Abtrennung vom Kohlenwasserstoff erhaltene Propen/Propan-Gemisch vor der Rückführung des Propens in den Oxidationsprozess in einem C₃-Splitter in Propen und Propan aufgetrennt wird.

Die Oxidation von Olefinen mit Hydroperoxiden zu Oxiranen ist bekannt und kann nach bekannten Methoden durchgeführt werden. Solche Methoden sowie ein technisches Verfahren werden beispielsweise in der WO 00/07965 beschrieben.

Zur Abtrennung der bei der Oxidation entstehenden Oxirane aus den Reaktionsgemischen können beispielsweise Destillationskolonnen verwendet werden. Hierbei werden Abgasströme am Kopf der Kolonnen erhalten. Diese Abgasströme enthalten als Komponenten stets nicht umgesetztes Olefin und eine geringe Menge Sauerstoff, der aus der Zersetzungsreaktion des eingesetzten Hydroperoxides stammt. Zur besseren Regelung der Destillation werden üblicherweise Inertgase, vorzugsweise Stickstoff, verwendet. Da diese gleichfalls über den Kopf der Kolonnen entnommen werden, enthalten die Abgasströme somit auch diese Gase. Damit ist es beim erfindungsgemäßen Verfahren nicht mehr notwendig, ein zusätzliches Gas zur Vermeidung explosionsfähiger Gemische in die Absorptionsanlage einzuspeisen.

Des Weiteren besitzen die als Absorptionsmittel eingesetzten Kohlenwasserstoffe ein ausgezeichnetes Lösungsvermögen für Olefine, so dass das Verfahren unter Verwendung vergleichsweise geringer Mengen Absorptionsmittel betrieben werden kann. Für die technische Anwendung ist das neue Verfahren deshalb außerordentlich vorteilhaft.

Der Begriff Kohlenwasserstoffe umfasst aliphatische, cyclische, alicyclische, gesättigte, ungesättigte und aromatische Kohlenwasserstoffe, die auch mit aliphatischen Resten substituiert sein können. Die Kohlenwasserstoffe können auch in Form ihrer Gemische in das erfindungsgemäße Verfahren eingesetzt werden. Es ist bevorzugt, dass die Kohlenwasserstoffe mehr als 10 Kohlenstoffatome im Molekül enthalten.

Vorzugsweise wird als Kohlenwasserstoff Tetradekan eingesetzt.

Der Begriff Tetradekan umfasst dabei ein Gemisch langkettiger Kohlenwasserstoffe der allgemeinen Formel CₙH₂ₙ₊₂, wobei n eine ganzzahlige Zahl zwischen 10 und 20, vorzugsweise zwischen 13 und 15 bedeutet. In diesem Gemisch soll die Komponente Tetradekan der Formel C₁₄M₃₀ anteilsmäßig in Mengen von vorzugsweise mindestens 10 Gew.-%, mehr bevorzugt mindestens 30 %, insbesondere mindestens 50 Gew.-% vorhanden sein, wobei die Gesamtmenge aller im Gemisch vorhandenen Komponenten 100 Gew.-% beträgt. Demzufolge ist es nicht erforderlich, dass das für das erfindungsgemäße Verfahren verwendete Tetradekan in besonders hoher Reinheit vorliegt. Besagtes Gemisch kann beispielsweise bei der Erdölraffination erhalten werden, wobei das Gemisch der entsprechenden Fraktion entnommen wird. Als weitere Komponenten können demzufolge neben dem Tetradekan weitere gesättigte Kohlenwasserstoffe, die auch verzweigt oder unverzweigt, langkettig, cyclisch oder alicyclisch sein können, vorliegen. Ein solches Gemisch kann auch ungesättigte Kohlenwasserstoffe oder aromatische Kohlenwasserstoffe enthalten.

Der zur Absorption verwendete Kohlenwasserstoff bzw. das zur Absorption verwendete Kohlenwasserstoffgemisch, insbesondere Tetradekan oder ein Kohlenwasserstoffgemisch enthaltend Tetradekan, weist vorzugsweise einen Siedepunkt zwischen 200 und 300 °C, vorzugsweise 220 bis 270 °C auf. Ein solcher Kohlenwasserstoff bzw. ein solches Kohlenwasserstoffgemisch zeigt ein außerordentlich günstiges Lösungsvermögen für die bei der Olefinoxidation mit Hydroperoxiden eingesetzten Olefine, insbesondere für Propen. Insbesondere Tetradekan oder Kohlenwasserstoffgemische enthaltend Tetradekan zeigen dieses vorteilhafte Lösungsvermögen.

Zur Durchführung des erfindungsgemäßen Verfahrens wird nun zweckmäßigerweise der aus einem Oxidationsprozess eines Olefins zu einem Oxiran (Epoxidation) stammende Abgasstrom, der auch eine Kombination mehrerer Abgasströme darstellen kann, mittels eines Verdichters auf einen Druck von 2 bis 10 bar, vorzugsweise 3 bis 6 bar, komprimiert, anschließend mit Kaltwasser auf eine Temperatur von vorzugsweise 5 bis 35 °C gekühlt, und zur Abtrennung des Olefins einer Absorptionsanlage zugeführt.

Solche Absorptionsanlagen bestehen vorzugsweise aus dem Absorptions- und einem nachgeschalteten Desorptionsapparat. Endprodukte einer solchen Anlage sind stets das durch selektive Absorption abgetrennte Gas sowie das Restgasgemisch. Außerdem fällt das regenerierte Absorptionsmittel an, das im Kreislauf in den Absorptionsapparat zurückgeführt wird.

In der Absorptionsanlage können vorzugsweise im Gegenstrom arbeitende Füllkörper-, Bodenkolonnen und Blasensäulen sowie in Sonderfällen Venturiwäscher eingesetzt werden. In der Gesamtanlage sind die Absorptions- und Desorptionsapparate zu einer kontinuierlich arbeitenden Einheit verbunden.

Dabei werden der Abgasstrom aus dem Oxidationssprozess zunächst dem Absorptionsapparat vorzugsweise im unteren Teil und der Kohlenwasserstoff im oberen Teil zugeführt. Dadurch kommt es zu einem ausgeprägten Gegenstrom der Ströme. Eine weitere Zuführung von zusätzlichem Inertgas, wie es beim Stand der Technik geschilderten Verfahren erforderlich ist, ist nicht notwendig, da der Abgasstrom bereits aus dem Prozess Komponenten, vorzugsweise Stickstoff, enthält, die die Ausbildung einer explosionsfähigen Atmosphäre verhindern.

Der im Absorptionsapparat herrschende Druck von 2 bis 10 bar, vorzugsweise 3 bis 6 bar, erhöht die Löslichkeit der lösbaren Bestandteile des Abgasstroms, insbesondere des nicht umgesetzten Olefins, im Kohlenwasserstoff, der vorzugsweise Tetradekan ist. Am Kopf der Kolonne verlässt dann der reine, nicht lösbare Gasbestandteil den Absorptionsapparat. Dies sind vorzugsweise Stickstoff, Sauerstoff und in geringen Mengen Olefin, beispielsweise Propen, falls dieses in den Oxidationsprozess eingesetzt wird.

Durch den Anteil an inerten Gasen im Abgasstrom der Oxidation verläuft die Stofftrennung außerhalb des Bereiches, in welchem sich explosionsfähige Gemische mit Sauerstoff bilden können. Deshalb erübrigt sich eine zusätzliche Einspeisung weiterer Gase zur Ausbildung nicht explosionsfähiger Gemische. Dieser nicht lösbare Gasanteil kann beispielsweise einer Verbrennung zugeführt werden.

Der am Kolonnensumpf anfallende und mit Olefin beladene Kohlenwasserstoff wird der Desorptionsstufe zugeführt. Für die Desorption stehen zwei Möglichkeiten zur Verfügung, die nahezu äquivalent sind und deren Einsatz von der Verfügbarkeit einer Kälteanlage abhängt.

Steht eine Kälteanlage mit Sole (ca. - 35 °C) zur Verfügung, wird die Desorption vorzugsweise in einer Destillationskolonne bei einem Druck von 1 bis 3 bar durchgeführt. In diesem Fall wird das Olefin in der Kolonne vom Kohlenwasserstoff gereinigt und über den Kopf der Kolonne in flüssiger Form abgetrennt, mit einer herkömmlichen Pumpe als Flüssigkeit auf ein höheres Druckniveau gebracht und entweder direkt der Reaktionsstufe oder einer weiteren Reinigungsstufe zugeführt. Am Sumpf fällt der vom Olefin befreite Kohlenwasserstoff an, welcher abgekühlt und anschließend in die Absorptionskolonne zurückgeführt wird.

Steht keine Kälteanlage zur Verfügung, so empfiehlt es sich, die Desorption als einstufige Entspannungsverdampfung, auch als Flash bezeichnet, bei einem Druck von 1 bis 3 bar und einer Temperatur von 50 bis 100 °C, vorzugsweise 70 bis 90 °C durchzuführen. In diesem Fall wird das Olefin dampfförmig abgetrennt und in einem anschließenden Wärmetauscher gekühlt, beispielsweise auf ca. 35 °C oder auf Raumtemperatur. Mit Hilfe eines Verdichters wird der abgekühlte Gasstrom auf einen Druck von ca. 11 bis 12 bar verdichtet, so dass er unter Verwendung von normalem Flusswasser als Kühlmittel verflüssigt werden kann. Als Verdichter eignen sich beispielsweise Hubkolbenverdichter. Der in der Verdampfung übrigbleibende Kohlenwasserstoff enthält in diesem Fall noch geringe Konzentrationen des Olefins. Wird Propen als Olefin eingesetzt, so beträgt der Anteil ca. Gew.-1 %. Er wird in einem Wärmetauscher abgekühlt und in die Absorptionsstufe zurückgeführt. Der verflüssigte Gasstrom kann nun entweder direkt der Reaktionsstufe oder einer weiteren Reinigungsstufe zugeführt werden.

Demgemäß ist das erfindungsgemäße Verfahren in einer bevorzugten Ausführungsform **dadurch gekennzeichnet, dass** das Olefin bei einem Druck von 3 bis 6 bar und einer Temperatur von 5 bis 35 °C absorbiert und bei einem Druck von 1 bis 3 bar in einer Destillationskolonne über Kopf flüssig oder in einer Entspannungsverdampfung bei einem Druck von 1 bis 3 bar und einer Temperatur von 70 bis 90°C dampfförmig abgetrennt wird.

In einer weiteren Ausführung ist es möglich, die Absorptionsanlage auch in Form einer Extraktionsanlage zu betreiben. Dies ist insbesondere dann von Interesse, wenn das abzutrennende Olefin beim Entspannen der Apparatur aus dem Kohlenwasserstoff nicht gasförmig desorbiert werden kann, sondern sich beispielsweise als flüssige Phase abscheidet. Geeignete Anlagen bestehen beispielsweise aus einer Gegenstromextraktionsapparatur mit nachgeschalteten Rektifizierkolonnen zur Aufbereitung des Kohlenwasserstoffs und des flüssigen Olefins.

Das Olefin, das vorzugsweise in einer Reinheit von mindestens 95 % anfällt, kann nun ohne weitere Reinigungsschritte dem Oxidationsprozess mit dem Hydroperoxid erneut zugeführt werden.

Besonders vorteilhaft beim erfindungsgemäßen Verfahrens ist, dass das Olefin in dem Maße, wie es aus dem Abgasstrom der Oxidation abgetrennt wird, wieder in den Oxidationsprozess zurückgeführt werden kann. Somit ist ein wirtschaftlich außerordentlich günstiges und kontinuierlich zu betreibendes Verfahren möglich.

Der Abgasstrom umfaßt zusätzlich zum Olefin noch weitere Komponenten, nämlich gesättigte Kohlenwasserstoffe, die bereits im eingesetzten Olefin enthalten sind.

Erfindungsgemäß wird in die Oxidationsstufe als Olefin Propen eingesetzt. Dieses enthält als gesättigten Kohlenwasserstoff Propan. Vorzugsweise können Fraktionen eingesetzt werden, die Propen und Propan im Volumenverhältnis von etwa 97 : 3 bis 95 : 5 enthalten. Solche Gemische werden auch als Propen mit der Reinheitsstufe "chemical grade" bezeichnet. Vorzugsweise wird bei der Propenoxidherstellung Propen dieser Reinheitsstufe verwendet.

Der Abgasstrom aus der Umsetzung von Propen zu Propenoxid enthält daher als Olefin Propen sowie als gesättigten Kohlenwasserstoff Propan.

Wie vorstehend erläutert umfasst dann der Abgasstrom neben dem Propen und Propan auch Inertgase, insbesondere Stickstoff, und eine geringe Menge Sauerstoff.

Im erfindungsgemäßen Verfahren wird das vom Propen und Propan befreite Abgas am Kopf des Absorptionsapparates und der mit Propen und Propan beladene Kohlenwasserstoff, welcher vorzugsweise Tetradekan ist, am Sumpf der Kolonne abgetrennt.

In der nachgeschalteten Desorptionsstufe wird nun bei einem Druck von ca. 1 bis 3 bar das Gemisch aus Propen und Propan nach einer der vorstehend beschriebenen Ausführungen entweder in flüssiger oder dampfförmiger Form vom Kohlenwasserstoff abgetrennt. Der Kohlenwasserstoff wird gekühlt und in die Absorptionsstufe zurückgeführt.

Der anfallende Strom der leichtsiedenden Komponenten Propen und Propan wird anschließend in einem C₃-Splitter, wie er beispielsweise beschrieben ist in Ullmann's Encyclopedia of Industrial Chemistry, 5. Ed., Vol A22, Seite 214, in die Komponenten Propen und Propan getrennt. Die Trennung kann dabei in einer Kolonne bei einem Druck von ca. 15 bis 25 bar erfolgen. Man kann zur Trennung auch thermisch gekoppelte Kolonnen verwenden und diese beispielsweise bei einem Druck von ca. 15 bzw. 25 bar betreiben. Das Propen wird dabei über den Kopf des als Kolonne ausgerüsteten C₃-Splitter abgezogen, das Propan über den Sumpf.

Demzufolge ist das erfindungsgemäße Verfahren auch **dadurch gekennzeichnet, dass** das nach der Abtrennung vom Kohlenwasserstoff erhaltene Propen/Propan-Gemisch in einem C₃-Splitter in Propen und Propan aufgetrennt wird.

Das abgetrennte Propen wird nun wieder dem Oxidationsprozess mit dem Hydroperoxid zugeführt. Das Propan kann als Energiequelle zur Dampferzeugung verwendet werden.

Als Hydroperoxide kommen für die Oxidation sämtliche aus dem Stand der Technik bekannten Hydroperoxide, die für die Umsetzung des Olefins geeignet sind, in Betracht. Beispiele für solche Hydroperoxide sind etwa tert.-Butylhydroperoxid oder Ethylbenzolhydroperoxid. Als Hydroperoxid kann auch Wasserstoffperoxid eingesetzt werden, beispielsweise als wässerige Lösung.

Die Abgasströme können dabei auch aus Oxidationsprozessen stammen, bei denen die Umsetzung des Olefins mit dem Hydroperoxid katalysiert wird, beispielsweise durch heterogene Katalysatoren.

In einer besonders bevorzugten Ausführung des erfindungsgemäßen Verfahrens wird Propen aus einem Abgasstrom abgetrennt, der bei der Oxidation von Propen mit Wasserstoffperoxid zu Propenoxid erhalten wird. Als bevorzugter Kohlenwasserstoff wird für diese Abtrennung Tetradekan eingesetzt.

Ebenso betrifft die Erfindung eine Vorrichtung zur Durchführung eines Verfahrens zur kontinuierlichen Rückführung des bei der Oxidation von Propen, welches als gesättigten Kohlenwasserstoff Propan enthält, mit Hydroperoxid zu Propenoxid nicht umgesetzten Propens, das im während der Oxidation entstehenden Abgasstrom enthalten ist, welcher Propan enthält, **dadurch gekennzeichnet, dass** die Vorrichtung wenigstens einen Reaktor zur Herstellung des Propenoxids, wenigstens eine Absorptions- und Desorptionseinheit zur Abtrennung des Propens und einen C₃-Splitter umfasst, wobei in der Absorptionseinheit Propen und Propan aus dem Abgasstrom durch Absorption in einem Kohlenwasserstoff abgetrennt werden, wobei in der Desorptionseinheit das Propen und Propan aus dem Kohlenwasserstoff desorbiert werden und wobei in dem C₃-Splitter die Komponenten Propen und Propan getrennt werden.

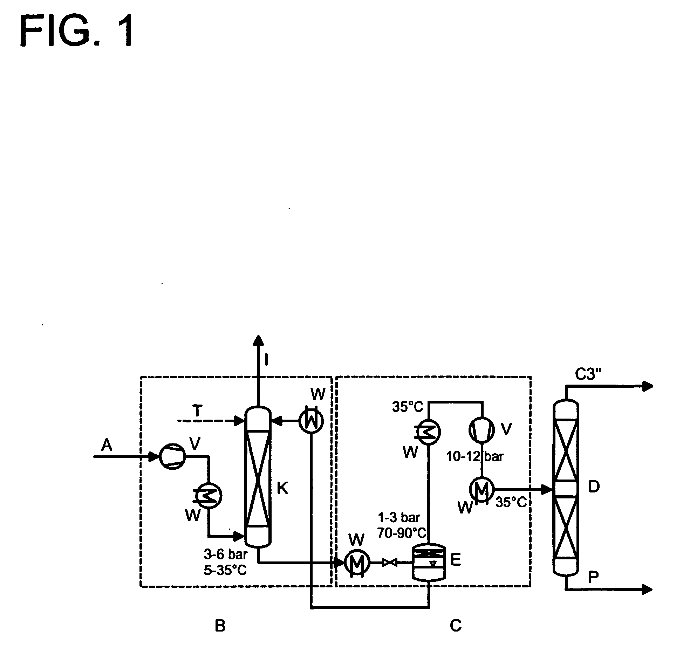

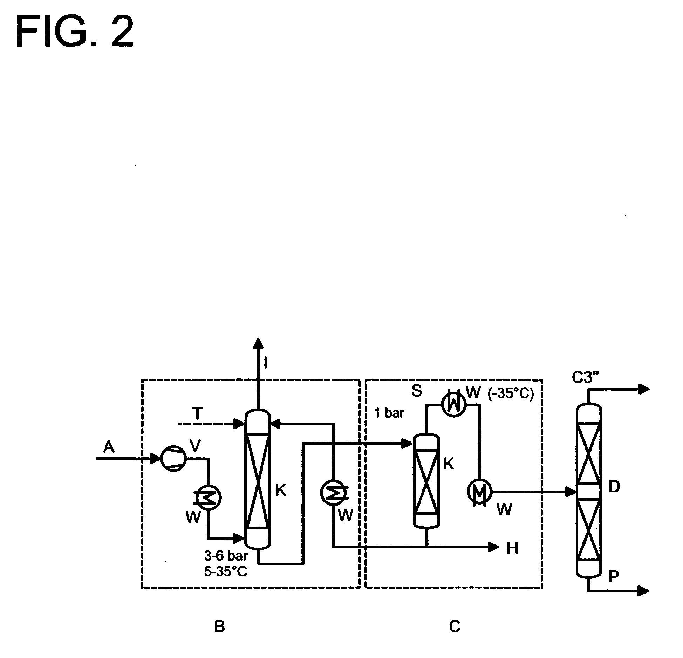

Die in den Figuren 1 und 2 dargestellten Fließbilder verdeutlichen, wie Propen mittels Tetradekanwäsche aus einem Abgasstrom, der bei der Epoxidierung (Oxidation) von Propen zu Propenoxid entsteht, erfindungsgemäß zurückgewonnen werden kann. Dabei ist in Figur 1 die Rückgewinnung ohne Kälteanlage und in Figur 2 die Rückgewinnung bei Verfügbarkeit einer Kälteanlage dargestellt.

### Bezugszeichenliste für die Figurenbeschreibung

- Figur 1: A: Abgas aus Epoxidierung
B Absorptionseinheit
V Verdichter
W Wärmetauscher
K Kolonne
T Tetradekan
I Inertgase (N₂), O₂
C Desorptionseinheit
V Verdichter
E Behälter für Entspannungsverdampfung
W Wärmetauscher
D C3-Splitter
P Propan (und Hochsieder)
C3" Propen "chemical grade"
- Figur 2: A: Abgas aus Epoxidierung
B Absorptionseinheit
V Verdichter
W Wärmetauscher
T Tetradekan
K Kolonne
I Inertgase (N₂) O₂
C Desorptionseinheit
S Kühlsole (-35 °C)
W Wärmetauscher
H Hochsieder
K Kolonne
D C3-Splitter
P Propan
C3" Propen "chemical grade"

## Patentansprüche

1. Verfahren zur kontinuierlichen Rückführung des bei der Oxidation von Propen, welches als gesättigten Kohlenwasserstoff Propan enthält, mit Hydroperoxid zu Propenoxid nicht umgesetzten Propens, das im während der Oxidation entstehenden Abgasstrom enthalten ist, welcher Propan enthält, **dadurch gekennzeichnet, dass** es die Stufen (i) bis (iii) umfasst
(i) Abtrennung des Propens und Propans aus dem Abgasstrom durch Absorption in einem Kohlenwasserstoff,
(ii) Desorption des Propens und Propans aus dem Kohlenwasserstoff, wobei das Gemisch aus Propen und Propan bei einem Druck von 1 bis 3 bar in einer Destillationskolonne in flüssiger Form oder in einer Entspannungsverdampfung bei einem Druck von 1 bis 3 bar und einer Temperatur von 50 bis 100 °C in dampfförmiger Form abgetrennt wird;
(iii) Rückführung des in Stufe (ii) erhaltenen Propens in den Oxidationsprozess,
wobei das nach der Abtrennung vom Kohlenwasserstoff erhaltene Propen/ Propan-Gemisch vor der Rückführung des Propens in den Oxidationsprozess in einem C₃-Splitter in Propen und Propan aufgetrennt wird.

2. Verfahren nach Anspruch 1, **dadurch gekennzeichnet, dass** der in Stufe (ii) nach Desorption des Olefins erhaltene Kohlenwasserstoff in Stufe (i) zurückgeführt wird.

3. Verfahren nach Anspruch 1 oder 2, **dadurch gekennzeichnet, dass** als Kohlenwasserstoff Tetradekan eingesetzt wird.

4. Verfahren nach einem der Ansprüche 1 bis 3, **dadurch gekennzeichnet, dass** das Propen bei einem Druck von 3 bis 6 bar und einer Temperatur von 5 bis 35 °C absorbiert wird.

5. Verfahren nach einem der Ansprüche 1 bis 4, **dadurch gekennzeichnet, dass** der Abgasstrom Inertgase und eine geringe Menge Sauerstoff umfasst.

6. Verfahren nach Anspruch 5, **dadurch gekennzeichnet, dass** der Abgasstrom Stickstoff umfasst.

7. Vorrichtung zur Durchführung eines Verfahrens zur kontinuierlichen Rückführung des bei der Oxidation von Propen, welches als gesättigten Kohlenwasserstoff Propan enthält, mit Hydroperoxid zu Propenoxid nicht umgesetzten Propens, das im während der Oxidation entstehenden Abgasstrom enthalten ist, welcher Propan enthält, **dadurch gekennzeichnet, dass** die Vorrichtung wenigstens einen Reaktor zur Herstellung des Propenoxids, wenigstens eine Absorptions- und Desorptionseinheit zur Abtrennung des Propens und einen C₃-Splitter umfasst, wobei in der Absorptionseinheit Propen und Propan aus dem Abgasstrom durch Absorption in einem Kohlenwasserstoff abgetrennt werden, wobei in der Desorptionseinheit das Propen und Propan aus dem Kohlenwasserstoff desorbiert werden und wobei in dem C₃-Splitter die Komponenten Propen und Propan getrennt werden.

## Claims

1. A process for the continuous recirculation of the propene which has not been reacted in the oxidation of propene comprising propane as saturated hydrocarbon by means of hydrogen peroxide to give propene oxide and is comprised in the offgas stream which is formed during the oxidation and comprises propane, which comprises the steps (i) to (iii)
(i) separating the propene and propane from the offgas stream by absorption in a hydrocarbon,
(ii) desorbing the propene and propane from the hydrocarbon, with the mixture of propene and propane being separated off in liquid form at a pressure of from 1 to 3 bar in a distillation column or in gaseous form at a pressure of from 1 to 3 bar and a temperature of from 50 to 100°C in a flash evaporation,
(iii) recirculating the propene obtained in step (ii) to the oxidation process,
where the propene/propane mixture obtained after separation from the hydrocarbon is separated into propene and propane in a C₃ splitter before recirculating the propene to the oxidation process.

2. The process according to claim 1, wherein the hydrocarbon obtained after desorption of the olefin in step (ii) is recirculated to step (i).

3. The process according to claim 1 or 2, wherein the hydrocarbon used is tetradecane.

4. The process according to any of claims 1 to 3, wherein the propene is absorbed at a pressure of from 3 to 6 bar and a temperature of from 5 to 35°C.

5. The process according to any of claims 1 to 4, wherein the offgas stream comprises inert gases and a small amount of oxygen.

6. The process according to claim 5, wherein the offgas stream comprises nitrogen.

7. An apparatus for carrying out a process for the continuous recirculation of the propene which has not been reacted in the oxidation of propene comprising propane as saturated hydrocarbon by means of hydrogen peroxide to give propene oxide and is comprised in the offgas stream which is formed during the oxidation and comprises propane, in which the apparatus comprises at least one reactor for preparing the propene oxide, at least one absorption and desorption unit for separating off the propene and a C₃ splitter, where propene and propane are separated from the offgas stream by absorption in a hydrocarbon in the absorption unit, the propene and propane are desorbed from the hydrocarbon in the desorption unit and the components propene and propane are separated in the C₃ splitter.

## Revendications

1. Procédé de recyclage continu du propène non réagi lors de l'oxydation de propène, qui contient du propane en tant qu'hydrocarbure saturé, avec de l'hydroperoxyde en oxyde de propène, qui est contenu dans le courant de gaz d'échappement formé pendant l'oxydation, qui contient du propane, **caractérisé en ce qu'**il comprend les étapes (i) à (iii)
(i) séparation du propène et du propane du courant de gaz d'échappement par absorption dans un hydrocarbure,
(ii) désorption du propène et du propane de l'hydrocarbure, le mélange de propène et de propane étant séparé sous forme liquide à une pression de 1 à 3 bars dans une colonne de distillation ou sous forme gazeuse par évaporation flash à une pression de 1 à 3 bars et à une température de 50 à 100 °C ;
(iii) recyclage du propène obtenu à l'étape (ii) dans le procédé d'oxydation,
le mélange propène/propane obtenu après la séparation de l'hydrocarbure étant séparé en propène et propane dans un dispositif de séparation des composés en C₃ avant le recyclage du propène dans le procédé d'oxydation.

2. Procédé selon la revendication 1, **caractérisé en ce que** l'hydrocarbure obtenu à l'étape (ii) après désorption de l'oléfine est recyclé à l'étape (i).

3. Procédé selon la revendication 1 ou 2, **caractérisé en ce que** du tétradécane est utilisé en tant qu'hydrocarbure.

4. Procédé selon l'une quelconque des revendications 1 à 3, **caractérisé en ce que** le propène est absorbé à une pression de 3 à 6 bars et à une température de 5 à 35 °C.

5. Procédé selon l'une quelconque des revendications 1 à 4, **caractérisé en ce que** le courant de gaz d'échappement comprend des gaz inertes et une petite quantité d'oxygène.

6. Procédé .selon la revendication 5, **caractérisé en ce que** le courant de gaz d'échappement comprend de l'azote.

7. Dispositif pour la réalisation d'un procédé de recyclage continu du propène non réagi lors de l'oxydation de propène, qui contient du propane en tant qu'hydrocarbure saturé, avec de l'hydroperoxyde en oxyde de propène, qui est contenu dans le courant de gaz d'échappement formé pendant l'oxydation, qui contient du propane, **caractérisé en ce que** le dispositif comprend au moins un réacteur pour la fabrication de l'oxyde de propène, au moins une unité d'absorption et de désorption pour la séparation du propène et un dispositif de séparation des composés en C₃, le propène et le propane étant séparés du courant de gaz d'échappement dans l'unité d'absorption par absorption dans un hydrocarbure, le propène et le propane étant désorbés de l'hydrocarbure dans l'unité de désorption et les composants propène et propane étant séparés dans le dispositif de séparation des composés en C₃.
